# EUROPEAN PATENT SPECIFICATION

(11) **EP 3 753 524 B1**
(45) Date of publication and mention of the grant of the patent: **20.03.2024**
(21) Application number: 19181557.0
(22) Date of filing: 20.06.2019
(51) Int. Cl.: A61B 90/00

(54) **ULTRASOUND MARKER, ULTRASOUND MARKER SYSTEM AND METHOD OF OPERATING AN ULTRASOUND MARKER SYSTEM**
ULTRASCHALLMARKER, ULTRASCHALLMARKERSYSTEM UND VERFAHREN ZUM BETREIBEN EINES ULTRASCHALLMARKERSYSTEMS
MARQUEUR À ULTRASONS, SYSTÈME DE MARQUEUR À ULTRASONS ET PROCÉDÉ DE FONCTIONNEMENT D'UN SYSTÈME DE MARQUEUR À ULTRASONS

(43) Date of publication of application: 23.12.2020
(73) Proprietor: AURA Health Technologies GmbH, 10707 Berlin (DE)
(72) Inventor: RIEGER, Jan, 13187 Berlin (DE)
(74) Representative: Eisenführ Speiser

(56) References cited:
- WO-A1-2005/055849
- US-A1- 2018 055 476
- US-B2- 8 989 844
- US-B2- 10 034 655

## Description

The invention relates to an ultrasound marker that can be implanted in tissue and can be localized by means of an ultrasound probe. The invention further relates to an ultrasound marker system that comprises an ultrasound marker and a probe that is configured to localize the ultrasound marker. The invention also relates to a method of operating an ultra-sound marker system.

The present invention relates generally to medical imaging and surgery and more specifically to implantable markers, tags, transponders and to systems and methods for localizing such makers, tags and transponders within a patient's body e.g. during breast cancer diagnosis and therapy or e.g. during laparoscopy or in diagnostic and therapeutic procedures in general.

Medical imaging methods such as mammography, tomosynthesis or ultrasound imaging are used to identify and/or confirm the location of a lesion before a biopsy or a surgical procedure (e.g. lumpectomy). Two types of marking strategies are then used to mark the place of biopsy or the location of the lesion. After a biopsy procedure a biopsy marker (i.e. a small implantable object) is placed in the location of biopsy. The biopsy marker may remain implanted in the tissue for ever if the lesion is diagnosed as benign, or it may resorb after certain time or it may be removed during the following surgery, if the lesion is diagnosed a malignant. Prior to surgery a localization wire may be placed to mark the lesion, such that a tip of the wire is positioned in a defined relationship to the lesion (e.g. either in the center of it or at the border). Once the wire is placed, the patient may proceed to surgery to have a lumpectomy or biopsy performed. The surgeon then follows the wire to access and to remove the lesion.

One problem with using a wire for localization is that the wire may move between the time of placement and the surgical procedure. Consequently, the surgeon may misinterpret the location of the lesion. Additionally, in some cases the surgeon would prefer a different access path to the lesion than the localization wire dictates. Some surgeons are capable of making a 3D representation of the region of surgery in their mind based on 2D medical images. Only then, they are able to access the tip of the wire from a different angle rather than following the wire.

Alternatively, to localization wire, it has been suggested to mark the lesion with a radioactive seed. The radioactive seed is introduced by a needle into the lesion, the needle is withdrawn and the position of the seed is confirmed using mammography or ultrasound imaging. The surgeon then uses a radiosensitive handheld gamma probe to approximate the position of the radioactive seed. An initial incision may be made and the probe is then used to guide the surgery towards the seed and to remove the lesion with the radioactive seed inside.

One problem with using a radioactive seed for localization is that the seed might migrate within the patient's body between the time of placement and the surgical procedure. Thus, similarly to using a localization wire, the seed may not accurately identify the location of the lesion, particularly, since there is no external way to stabilize the seed once placed. Further, in many countries the regulatory hurdle for handling the radioactive material is too high to overcome and to employ this technique. Radioactive seeds are so small that they might be sucked up by a suction system and get lost. In some countries, losing one radioactive seed might result in losing the accreditation for handling radioactive material for the whole institution.

Alternatively, it has been proposed that other types of seeds such as magnetic, RF or RFID might be used to tag the lesion. The tags are delivered with a needle into the lesion. The surgeon then uses a compatible detector probe (magneto-detector, RF antenna etc.) to approximate the position and distance to the seed and to access and remove the lesion with it.

Similarly, to radioactive seeds, these seeds might migrate and not precisely identify the location of the lesion. Additionally, as electromagnetic fields are not homogeneous and not isotropic, it has been reported, that some of the seed's response is directionally dependent and thus they don't provide accurate navigation from all angles.

An echogenic marker that can emit ultrasound is known from US 10,034,655 B2.

One common disadvantage of the prior art techniques mentioned above is that the surgeon does not have any overview about the anatomy in the operating area around the marker. It has been suggested that an image guided surgery would lead to a higher precision.

It is an object of the invention as defined by claim 1 to provide an improved ultrasound marker. It is further object of the invention to provide an improved ultrasound marker system. It is further object of the invention to provide an improved ultrasound imaging system.

According to one aspect of the invention, the object is achieved by means of a marker that comprises an enclosure. The marker further comprises an energy transducer for transducing impeding sound energy into electric energy. The marker also comprises an ultrasound emitter that is configured to generate an ultrasound signal when fed with electric energy. The marker further comprises a trigger that is operatively connected to the ultrasound emitter and that can be activated via a wireless signal. The trigger is configured to control the generation of ultrasound by the ultrasound emitter in dependence of the wireless signal.

The invention includes the recognition that currently, if the surgeon wants to have image guidance for the surgery he has to alternate between ultrasound probe and detector probe. It would be useful if the detector probe was combined with the ultrasound probe which is one of the possible embodiments of the present invention.

An advantage of a marker as defined above is that the marker can be used in combination with a prior art hand-held ultrasound probe. In contrast to prior art passive ultrasound markers, the ultrasound marker according to the invention can be actively controlled by an additional wireless signal, for instance a light signal. The ultrasound marker according to the invention can convert impeding ultrasound energy into electric energy that in turn can be used to supply the ultrasound emitter. Generation of an ultrasound signal by the ultrasound emitter can be controlled by a light signal (or another wireless signal). Only when the trigger is activated, the ultrasound emitter will generate an ultrasound signal using electric energy that is generated by the energy transducer.

A marker according to the invention has the advantage that it can be easily used in combination with one or more further markers of the same type without effecting the ability to localize individual markers because only a markerthat is activated by an additional wireless signal will generate an ultrasound signal that can be localized by means of the probe while in prior art systems all passive ultrasound markers would backscatter impeding ultrasound energy and thus affect the localization of individual markers by means of an ultrasound probe.

Both, the energy transducer and the ultrasound emitter can comprise a piezo element. In particular, the energy transducer and the ultrasound emitter can be implemented by means of one single piezo element that serves for both transducing sound energy into electric energy and emitting ultrasound when supplied with electric energy.

Alternatively, the energy transducer and/or the ultrasound emitter can be implemented by means of a capacitive micromachined ultrasonic transducer (CMUT). The energy transducer may alternatively comprise a RC-resonance circuit, a RLC-resonance circuit, a LED-diode or the like.

In a preferred embodiment, the marker may additionally comprise an electric energy storage for instance a capacitor, that can store electric energy generated by the energy transducer. The electric energy storage preferably is connected to both, the energy transducer and the trigger that in turn is connected to the ultrasound emitter.

Additionally, the marker may comprise a battery or other type of energy storage.

The trigger is configured to respond to a light signal, preferably in infrared or near infrared spectrum. Accordingly, the wireless signal for activating the trigger preferably is a light signal in infrared or near infrared spectrum.

The trigger comprises a photosensitive element that preferably is sensitive to infrared light. The trigger can comprise a Field-Effect-Transistor (FET), a light diode and/or a phototransistor.

Additionally, an electrostatic discharge protection element (ESD element) may be provided.

Preferably, at least the energy transducer, the ultrasound emitter and optionally the electric energy storage are arranged within the enclosure.

The trigger or parts of the trigger may also be arranged within the enclosure. In a preferred embodiment, however, parts of the trigger, in particular one or more photodiodes or phototransistors are arranged outside the enclosure if the enclosure is made from an opaque material.

The marker may further comprise wires for electrically connecting elements of the marker within and/or outside the enclosure.

The marker may comprise an application specific integrated circuit (ASIC) or a complementary metal oxide semiconductor circuit (CMOS) that can for instance be used to modulate the emitted ultrasound signal with further information, such as sensory information.

The enclosure may enclose small elements that for instance are filled into a respective cavity of the enclosure. The small elements can be ultrasound microbubbles, microshells, polymer grains, glass bubbles, gas-filled cavities within a porous material or the like. Preferably, the small elements are configured to amplify, modulate and/or filter the ultrasound waves that reach the marker from the detector or the small elements may amplify, modulate and/or filter the ultrasound waves generated by the ultrasound emitter.

The marker may comprise fixation means that are configured to prevent dislocation of marker. Preferably the fixation means are part of the enclosure or external to it and provide fixation by anchoring the marker mechanically in soft tissue.

A marker comprising an enclosure, and energy transducer and/or an ultrasound emitter, for instance a piezo element, and having fixation means for anchoring the marker mechanically in soft tissue is an inventive concept of its own that can be implemented independently from further aspects and features mentioned in this description.

The enclosure may be made of biocompatible material or coated with biocompatible material.

The marker may further comprise an ultrasound resonator.

Preferably, the marker is configured to generate specific ultrasound waves.

The marker may be configured for permanent implantation, for instance in breast tissue or lymph nodes.

The marker may contain sensors to probe the physical and/or (bio-)chemical parameters of the surroundings of the marker such as temperature, oxygenation level, pH, etc. The measured data may be converted into electrical energy and modulated to the ultrasound signal emitted by the marker.

Preferably, the marker has a cross-section between 0.5 mm and 10 mm and preferably a length between 2 mm and 30 mm.

The marker preferably is visible in ultrasound imaging and/or in x-ray imaging, and/or in MRI imaging.

According to a further aspect, the object of the invention is achieved by a marker locating system that comprises an ultrasound marker, a handheld probe, a light source, a signal processor and a user guiding signal generator. The handheld probe comprises an ultra-sound transducer and is configured to emit and receive ultrasound. The signal processor is configured to process ultrasound signals received by the probe. "Processing ultrasound signals" means that the signal processor processes electric signals provided by the ultra-sound transducer of the handheld probe. The user guiding signal generator is configured to generate a user perceptible guiding signal in dependence of processed ultrasound signals.

Preferably, the probe is configured to emit ultrasound to energize the marker and it is configured to emit electromagnetic energy to trigger the ultrasound marker.

The signal processor preferably comprises a trained classifier. The trained classifier can be configured to determine a Baysian probability and may comprise a neural network.

The user guiding generator preferably is configured to generate a user perceivable feedback signal in dependence of processed ultrasound signals received by the probe. The user perceivable feedback signal can be an audible sound signal and/or visual feedback. Additionally or alternatively, user guiding signal generator can be configured to generate vibrations that can be sensed by the user.

The marker locating system may further comprise imaging means and a display.

The signal processor may implement supervised and unsupervised learning methods for clustering of relevant findings. The signal processor may comprise a number of classifiers that are trained with appropriate learning algorithms. In particular, the signal processor may combine different classifiers, in particular a combination of classifiers from a time domain and classifiers from a frequency domain.

The signal processor may be configured to
- produce final output for classification of findings;
- calculate the findings statistics based on Bayesian probability;
- determine the position of the finding by Bayesian probability; and
- provide feedback to the operator about the position.

The signal processor may comprise a feedforward multi-layer perceptron neural network trained by sequential backpropagation algorithm. The signal processor may implement kernel-based method to map feature vectors into higher-dimensional space and training an optimal hyper plane to fit data.

According to a further aspect of the invention a method for training supervised or unsupervised Computational Intelligence (CI) techniques for detection, review and diagnosis of clinical findings is provided that comprises the steps:
- localizing of an implantable marker in the diseased tissue;
- recording of the signals from diseased tissue in the close proximity of the implantable marker;
- recording of control signals from healthy tissue;
- clustering of the recordings using unsupervised learning method;
- training a number of classifiers with appropriate learning algorithms; and
- combining classifiers to produce final output for classification of findings

According to a further aspect of the invention a method for operating a marker locating system according comprises the steps of:
- emitting energy to the marker;
- receiving energy by the marker
- converting energy into electric energy in the marker
- storing the electric energy in the marker
- triggering switch and activating ultrasound emitter in the marker upon reception of a trigger signal received by the marker;
- emitting an ultrasound signal by the marker;
- receiving the ultrasound signal by the probe; and
- determining the distance between the marker and the probe based on ultra-sound signal received by the probe.

Further aspects of the invention are apparent from the exemplary embodiments illustrated in the Figures. Of the Figures
- Fig. 1:: shows a first embodiment of an ultrasound marker according to the invention;
- Fig. 2:: shows a marker locating system comprising an ultrasound marker according to the invention and a handheld ultrasound probe;
- Fig. 3:: shows a second embodiment of an ultrasound marker according to the invention;
- Fig. 4:: shows a third embodiment of an ultrasound marker according to the invention;
- Fig. 5:: shows a fourth embodiment of an ultrasound marker according to the invention;
- Fig. 6:: shows electric circuitry of an ultrasound marker according to the invention;
- Fig. 7:: shows an ultrasound marker having fixation means;
- Fig. 8:: shows a variant of an ultrasound maker according to the invention wherein the enclosure is filled with small elements;
- Fig. 9:: shows a variant of an ultrasound maker according to the invention wherein the enclosure is partially filled with small elements;
- Fig. 10:: illustrates the operation of a handheld probe in combination with implanted ultrasound markers;
- Fig. 11:: shows a detail of the handheld probe;
- Fig. 12:: illustrates information displayed on a display of the system in from Figure 2;
- Fig. 13:: Illustrates the operation of the handheld probe for localizing a marker;
- Fig. 14:: illustrates an output signal provided by an ultrasound transducer of the handheld probe;
- Fig. 15:: illustrates another output signal provided by an ultrasound transducer of the handheld probe; and
- Fig. 16:: illustrates implantation of an ultrasound marker by means of an implantation instrument.

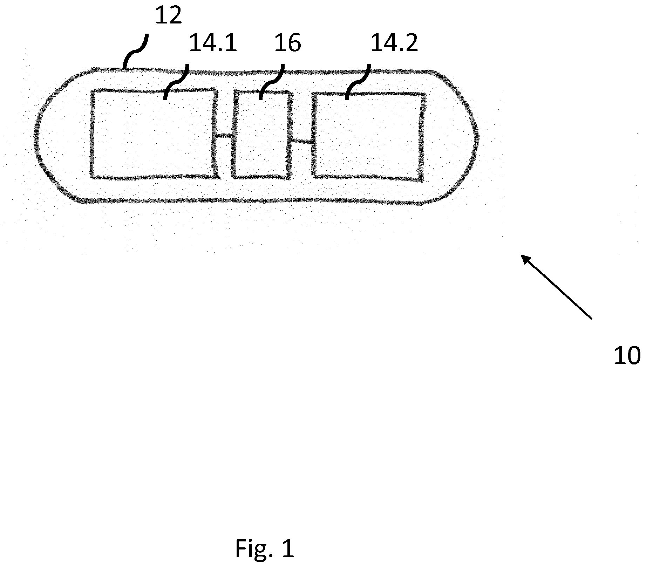

As can be taken from Figure 1, an ultrasound marker 10 comprises an enclosure 12. Within the enclosure, two piezo elements 14.1 and 14.2 are arranged. One piezo element 14.1 is configured to serve as energy transducer that transduces impeding sound energy into electric energy. The other piezo element is configured to serve an ultrasound emitter. Both piezo elements are operatively connected to a trigger 16 that comprises a photosensitive element. Trigger 16 is configured to be activated by a light signal, preferably an infrared or near-infrared light signal. Activating the trigger 16 causes the trigger to supply electric energy to the second piezo element 14.2 to thus cause the second piezo element 14.2 to generate an ultrasound signal. the energy supplied to the second piezo element 14.2 is generated by the first piezo element 14.1.

The enclosure 12 has a cross-section between 0.5 mm and 10 mm and a length between 2 mm and 30 mm depending on the intended placement within the patient's body. For the implantation within patient's breast or lymph nodes a cross-section between 0.5 mm and 2 mm and a length between 3 mm and 15 mm would be preferable. The enclosure 12 can be solid, or it is hollow and may be filled with gas, liquid, gel, hydrogel or other substances that fully or partially fill the space between the electronics and the inner surface of the hollow enclosure 12. The enclosure 12 may be formed from one or multiple biocompatible materials, e.g. stainless steel, Nitinol, glass, ceramics, plastic, epoxy. The enclosure 12 may also be formed from a non-biocompatible material and have a biocompatible coating, e.g. parylene, polyimide, silicone nitride, silicon dioxide, etc.

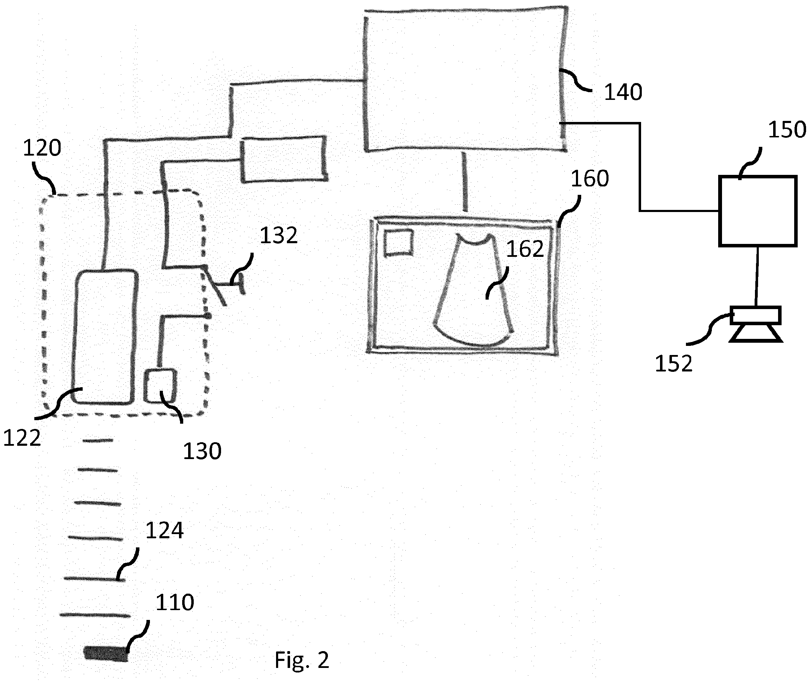

The marker location system 100 illustrated in Figure 2 comprises an ultrasound marker 110, a handheld probe 120, a trigger (light) source 130, a signal processor 140 and a user guiding signal generator 150. The handheld probe 120 comprises an ultrasound transducer 122 and is configured to emit and receive ultrasound. The signal processor 140 is configured to process electric signals. The user guiding signal generator 150 is configured to generate a user perceptible guiding signal in dependence of processed ultrasound signals. Preferably, the user guiding signal generator 150 comprises a speaker 152, i.e. an acoustic transducer for generating sound signals in response to electric signals fed to the acoustic transducer. The trigger (light) source 130 can be an integral part of the handheld probe 120 or it can be a separate component of the marker locating system 100. The trigger (light) source is connected to a trigger (light) source switch 132 for switching the trigger (light) source on or off, respectively. The switch 132 is a push button that activates the trigger (light) source 130 if and as long as pushed.

The marker 110 used in the marker locating system 100 can be of various types. In one embodiment, the marker 110 corresponds to the marker 10 as shown in Figure 1.

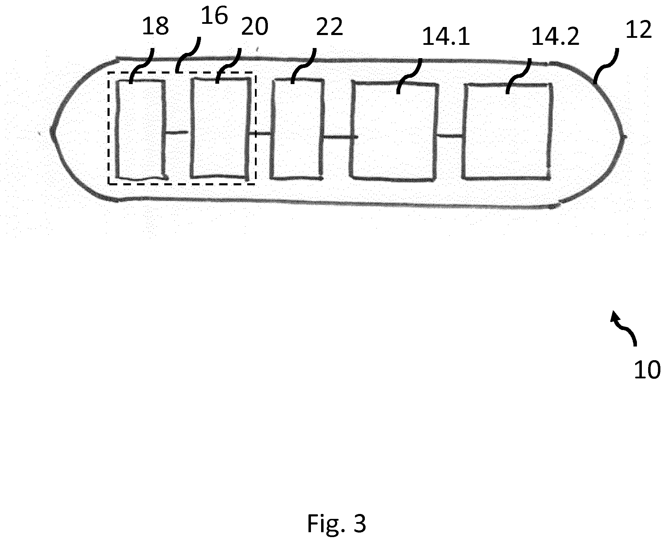

Alternatively, the marker 110 may correspond to one of the markers as shown in Figures 3 to 5.

Figure 3 illustrates a markerthat comprises two piezo elements 14.1 and 14.2 and a trigger 16 as the marker shown in Figure 1. In addition to what is shown in Figure 1, the marker 10 as depicted in Figure 3 exhibits further detail. In particular, trigger 16 comprises a photosensitive element 18 and a switch 20. Switch 20 can be activated by the photosensitive element 18. That means switch 20 is switched in its "on"-state if light impedes the photosensitive element 18. Trigger 16 and switch 20 in particular is operatively connected to the first piezo element 14.1 via an electrostatic discharge protection element 22. The electrostatic discharge protection element 22 can be implement by way of a Zener-diode. Modern breast surgery often involves the use of electro-cutting tools, electrocautery tools, and/or other tools which can generate electrical pulses of more than 1 kV. This might destroy the trigger 16. The can be prevented by means of the electrostatic discharge protection element 22.

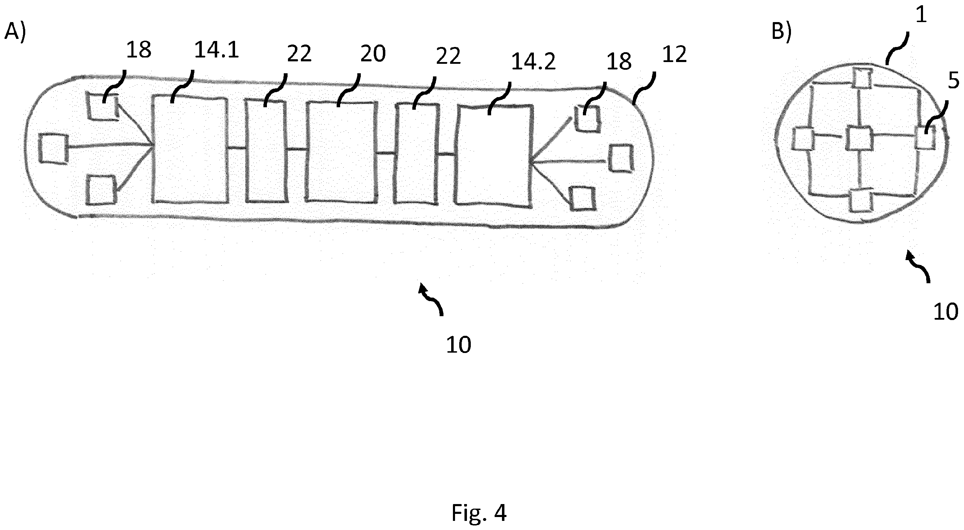

The photosensitive element 18, for instance a photodiode or the like, can be arranged within the enclosure 12 as shown in Figure 3 and 4. If the photosensitive element 18 is arranged within the enclosure 12, at least part of the enclosure 12 should be made of transparent material e.g. glass, that is transparent for the particular light wavelength so that the light can penetrate to the component and that light can impede on the photosensitive element 18 within the enclosure 12.

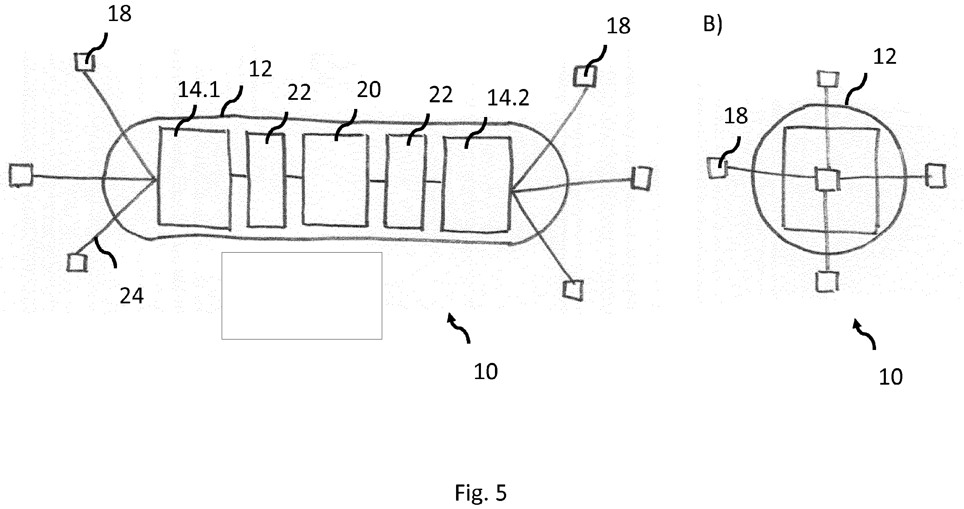

Alternatively, the photosensitive element 18 can be arranged outside enclosure 12 and would be connected to the components inside enclosure 12 by means of wires 24. An example for an ultrasound marker 10 having photosensitive elements 18 arranged outside enclosure 12 is shown in Figure 5.

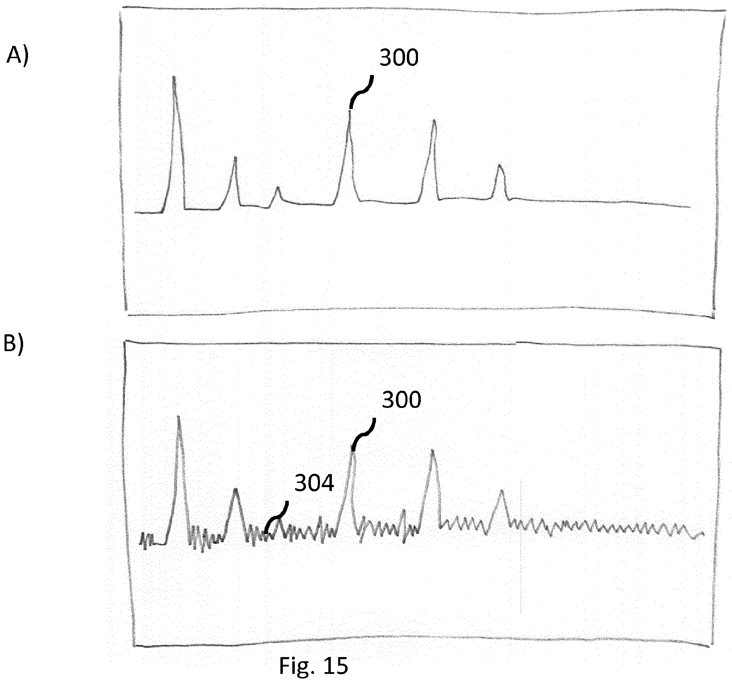

In general, a part of the electronics of a marker, e.g. the photosensitive elements 18, may be arranged outside of the enclosure 12 as illustrated in Figures 5 A and 5 B. The electronics outside of the enclosure 12 may be then connected to the electronics inside of the enclosure 12 by wires 24 that may be formed from elastic or superelastic material and/or from shape memory material, e.g. Nitinol or stainless steel, such that the wires 24 take a predetermined shape after deployment within tissue to prevent migration. The wires 24 may however be elastically compressed to minimize the size of the marker 10 in e.g. a delivery device (cf. Figure 15). The wires 24 may also be rigid. In one exemplary embodiment the wires may also act as antennas.

Optionally, instead of electrical components the wires 24 may carry other components that facilitate the visibility of the marker in biomedical imaging.

As can be taken from Figures 4 and 5, preferably a plurality of photosensitive elements 18 are provided so that the ultrasound marker 10 can respond to impeding light from any direction. In such a preferred embodiment, the light can reach the light sensitive component, e.g. the photosensitive diode 18, from all directions so that the marker 10 can be activated in an arbitrary orientation towards the probe 120. In some embodiments there are multiple light sensitive components 18 to facilitate that.

Preferably, photosensitive elements 18 are sensitive for infrared or near-infrared light. Using infrared light, for instance infrared light having a wavelength between 800 nm and 950 nm, has the advantage that infrared light is exposed to less scattering in body tissue. Accordingly, a single ultrasound marker 10 can be selectively activated by a directed light ray without activating neighbouring ultrasound marker elements.

In one embodiment there is one single piezo transceiver 14 that converts incoming ultra-sound waves into a voltage, then there is a photosensitive element 18 that controls a switch 20. If the switch 20 is closed the generated voltage is fed back to the single piezo transceiver 14 and modulates its response to incoming ultrasound waves so that background noise signal is reflected towards the probe 120 and is detected by the signal processor 140.

In an alternative embodiment there is one single piezo transceiver 14 that converts incoming ultrasound waves into a voltage, then there is a photosensitive element 18 that controls a switch 20 or a field effect transistor (FET). If the switch 20 is closed the impedance on the single piezo transceiver 14 is changed and its response to incoming ultrasound waves changes so that if reflection signal is detected by the signal processor 140 and analysed then the triggered changes in the reflection can be found the signal.

In an alternative embodiment there is a first piezo transceiver 14.1 that converts incoming ultrasound waves into a voltage, then there is a photosensitive element 18 that controls a switch 20. If the switch 20 is closed the generated voltage is fed to a second piezo transceiver 14.2 that converts the voltage into a specific ultrasound signal that is transmitted towards the probe 120 and is detected by the signal processor 150.

In further embodiments the generated voltage may be stored, delayed, amplified and modulated by dedicated components of the marker in order to generate a specific background noise that can be received by the probe 120 and unmistakable recognized by the signal processor 150.

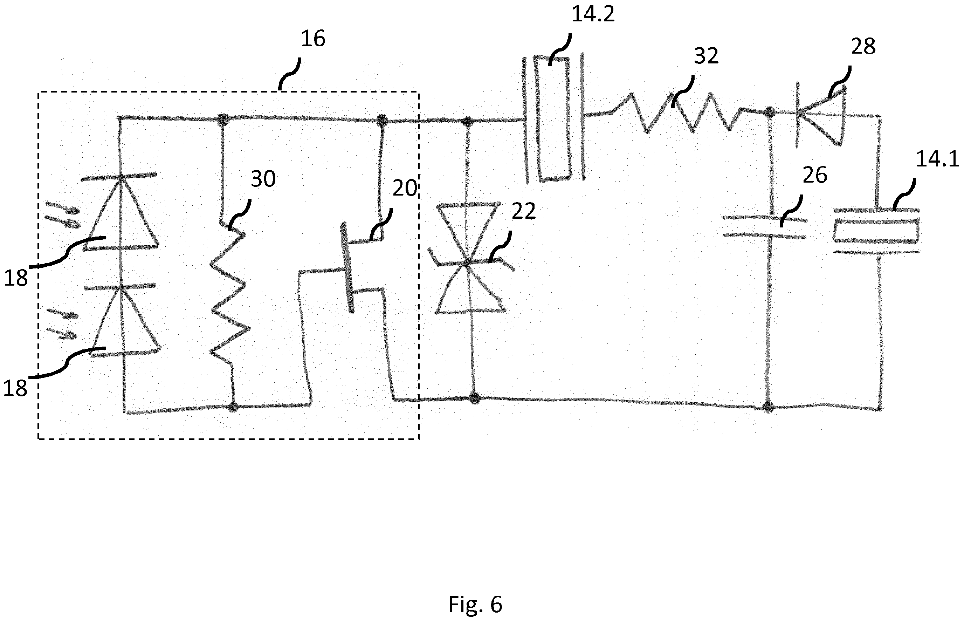

Electric circuitry that can be used to implement an ultrasound marker element 10 as shown in Figures 3 to 5 is depicted in Figure 6. As can be taken from Figure 6, the ultrasound marker element 10 may comprise an energy storage 26 that comprises a capacitor. For charging the capacitor, first the piezo element 14.1 is connected to the capacitor 26 via a Schottky diode 28 for rectifying electric current generated by the first piezo element 14.1 in response to impeding ultrasound.

A second piezo element 14.2 serves for generating and emitting ultrasound. For activating the second piezo element 14.2, trigger 16 is provided that comprises two photodiodes 18 that are a photosensitive element and a switch 20 that is implemented by means of a field effect transistor (FET). Gate wattage for switching the field effect transistor 20 into its conducting state is limited by means of a resistor 30. If infrared light impedes on photodiodes 18, field effect transistor 20 is switched into its conducting on-state, thus supplying the second piezo element 14.2 with electric power stored in condenser 26. In order to protect the second piezo element 14.2 a protection resistor 32 is switched in line with the second piezo element 14.2. Further, Zener-diode 22 is provided as an electrostatic discharge protection element.

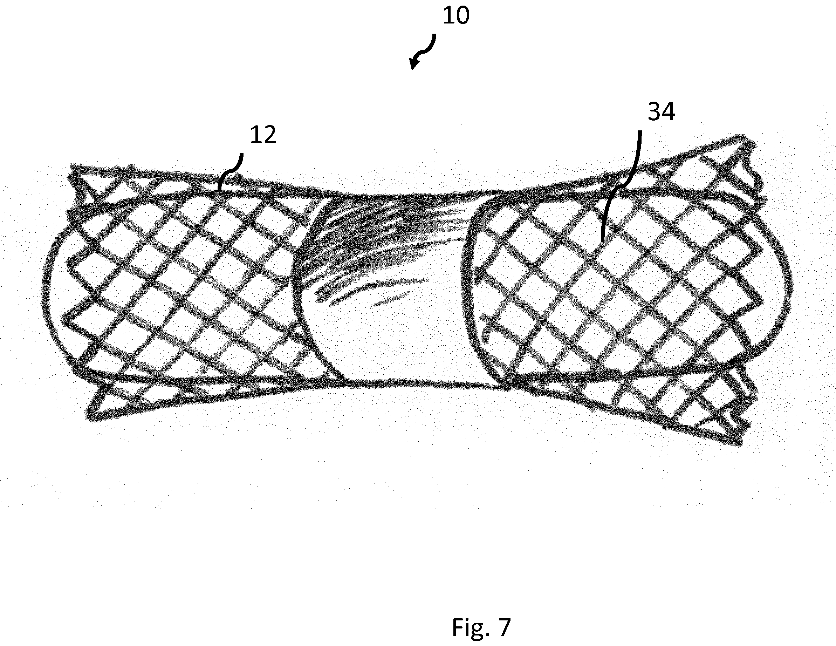

As shown in Figure 7 the ultrasound marker 10 can be provided with fixation means 34 that serves to prevent dislocation of the ultrasound marker 10 when implanted into body tissue. The fixation element 34 may for example be a mesh that is attached to the enclosure 12 of the ultrasound marker 10. The fixation means prevents the marker 10 from migrating within patient's body. The mesh 34 can be made formed from elastic or superelastic material and/or from shape memory material, e.g. Nitinol, stainless steel, polymer, such that the mesh 34 takes a predetermined shape after deployment within tissue to prevent migration. The mesh 34 may however be elastically compressed to minimize the size of the marker 10 in e.g. the delivery device.

In order to improve the acoustic properties of the ultrasound marker 10 for ultrasound, the enclosure 12 can be filled with small elements 36 such as microbubbles, microshells, polymergrains, glasbubbles, gas-filled cavities within a porous material, or the like. These small elements 36 are configured to amplify, modulate and/or filter the ultrasound waves generated by the ultrasound emitter 14.2. The small elements 36 may fill partially or fully the space between the electronics within the enclosure 12. The small elements 36 may amplify, modulate and/or filter the ultrasound waves generated by the piezo transceiver 14.2. The form of interaction with the ultrasound waves and the small elements 36 depends on the composition of the small elements 36. The small elements 36 may be formed by a flexible phospholipid membrane that encloses gas-filled cavity. The small elements 36 might then be compressed by the incident ultrasound waves and by the flexibility of the membrane the small elements may generate nonlinear response and higher harmonic resonances that may be transmitted towards the probe 120 and be detected by the signal processor 140. The small elements 36 may be formed by rigid, hollow and gas-filled silica shells. The small elements 36 might then burst under the incident ultrasound waves and may generate nonlinear response, Doppler or higher harmonic resonances or reflections that may be transmitted towards the probe 120 and be detected by the signal processor 140.

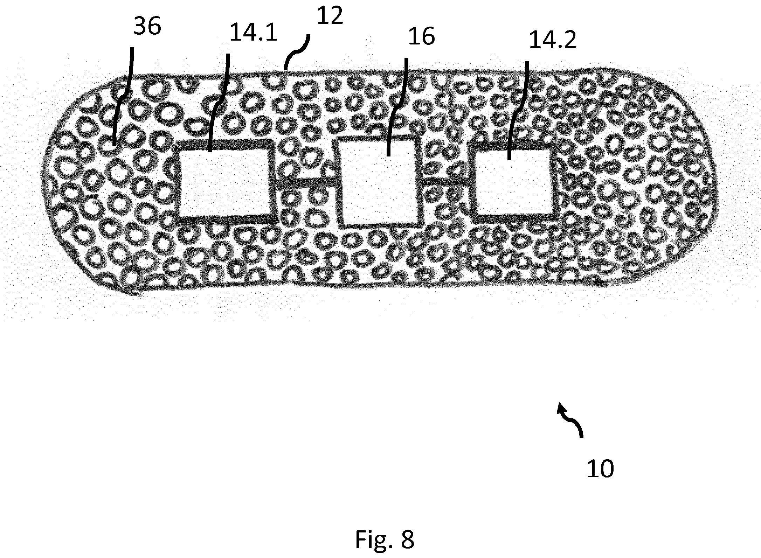

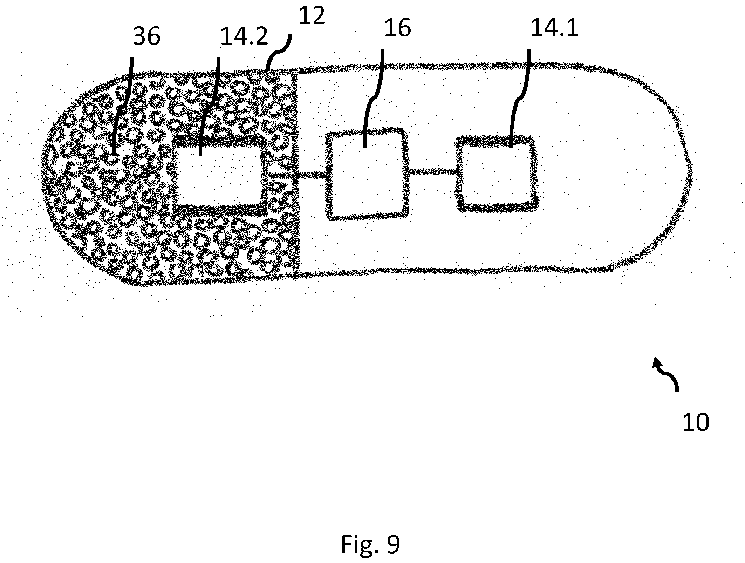

These small elements 36 may fill the entire free space within enclosure 12 of the ultrasound marker 10 as shown in Figure 8. Alternatively, small elements 36 may only be provided in a part surrounding the ultrasound emitter 14.2 within the enclosure 12 of the ultrasound marker 10 as shown in Figure 9.

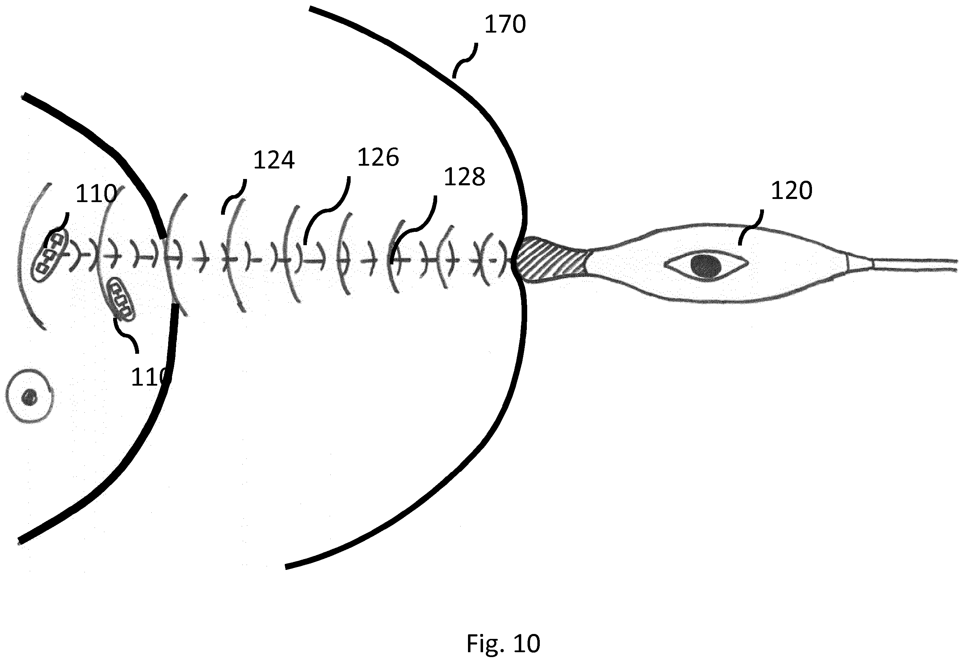

The operation of the ultrasound marker locating system shown in Figure 2 is illustrated in Figure 10. Handheld probe 120 can emit and receive ultrasound waves as in prior art handheld probes. Further, handheld probe 120 can emit a ray of infrared light 128. In Figure 10, the ultrasound wave emitted by handheld probe 120 is indicated by the reference sign 124. If the ultrasound waves 124 hit an ultrasound marker 110, ultrasound marker 110 reflects the ultrasound wave. However, all ultrasound marker elements that are implanted for example in a breast 170 of the patient will reflect a part of the impeding ultrasound wave because the ultrasound is scattered within the breast tissue. To selectively localize one specific ultrasound marker element 110, ultrasound marker element 110 can actively emit ultrasound that can be registered by handheld probe 120. As shown in Figures 1 and 3 to 5 above, the ultrasound marker element 110 comprises an ultrasound emitter 14.2 for actively generating an ultrasound signal. The generation of an ultrasound signal by the ultra-sound marker 110 is triggered by trigger 16 that can be activated by infrared light. Accordingly, the handheld probe 120 can emit an infrared light ray 128 to selectively cause an ultrasound marker element 110 to generate and emit an ultrasound signal that can be registered by the handheld probe 120.

In one embodiment the probe 120 is a hand-piece similar to a standard ultrasound probe that has a distal end that can be placed against patient's skin and that has a proximal end that can be hold by the user. The probe 120 has two functional units one being the light-source 130 and second being the ultrasound transducer 122.

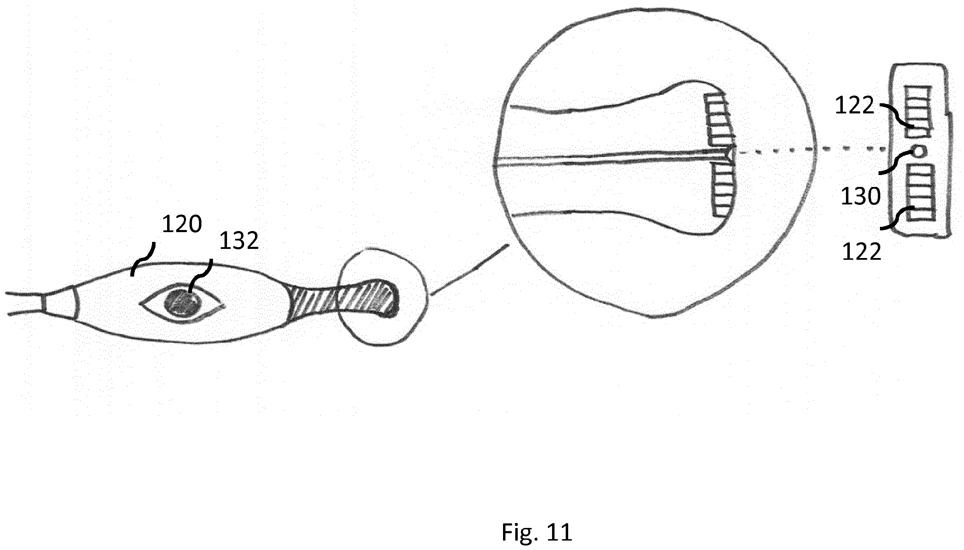

The ultrasound transducer 122 may be a single channel ultrasound transducer that only generates A-mode ultrasound signals or it may be an array of ultrasound transceivers 122 as shown in Figure 11 where the ultrasound transceivers 122 are arranged in a 1D or 2D array. The array may provide between 2 and 128 channels or more in order to generate a proper ultrasound image 162 that may be displayed on the display 160. Even if the probe 120 has more than one channel, displaying the ultrasound image 162 is only optional as the algorithm to find the marker may very well work only with radiofrequency (RF) data or channel data that is acquired by one or more channels of the ultrasound transducer 122. The RF data or channel are transmitted by wire or wirelessly from the transducer 122 to the signal processor 140. In one embodiment the signal processor 140 is separated from the ultrasound transducer 122. In yet another embodiment the signal processor 140 and the handheld probe 120 are combined to build one hand-held system which may be wired to power or may contain internal batteries to be able to operate without a power source. The probe 120 may be sterilizable or it may be suited to be used with a sterile sheath during the surgery. It may also be disposable fully or partially.

In a preferred embodiment the light-source 130 is part of the probe 120 and may be manually switched on and off by a switch 132 on the probe 120. The opening that emits the light may be in-between the ultrasound transceivers 122 as shown in Figure 11 or it may be placed sideways from the transceivers in order not to interrupt the continuity of collected ultrasound data for image construction. The light might be generated directly in the probe or the light source might be place in a separate unit and the light might be directed by the means of optical cable to the distal tip of the probe 120.

The user can activate the light source 130 by operating the switch 132. This is advantageous especially for systems where the ultrasound image is used for diagnostic purposes as well and the light source would only be activated once the localization of a marker 10 is desired.

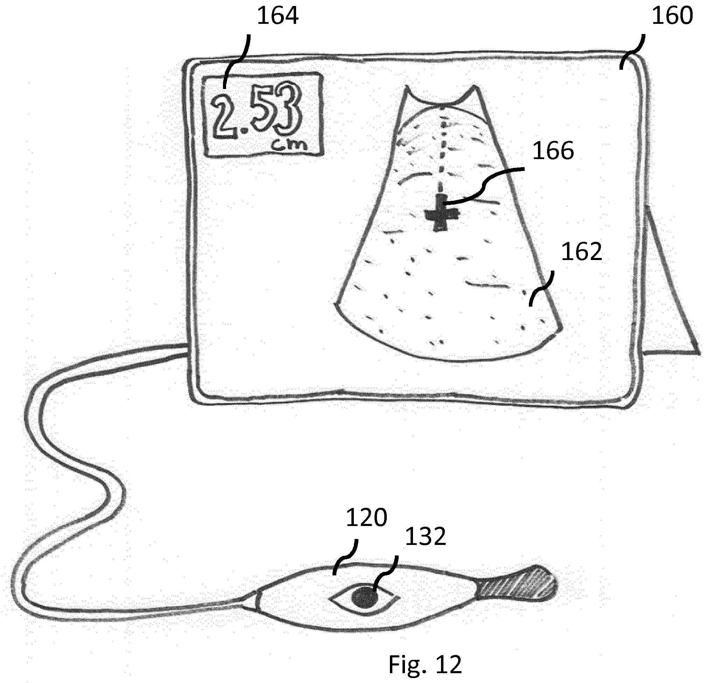

The signal processor 140 processes the output signal provided by the probe 120 and contains an algorithm to find the marker signal in the received output signal. Once the marker signal is found the signal processor 140 may calculate the distance of the marker 10 from the probe 120 and display it as a distance value 164 on the display 162 or it may also show a colour bar where a colour is assigned to particular depth. The signal processor 140 may calculate the position of the marker 10 within the ultrasound image 162 and depict it as an overlay 166 as shown in Figure 12.

In one embodiment, the computer system has a sound system 150 that can produce sound with a different pitch or intensity depending on the proximity of the marker 10 to the probe 120. Thus the user would be able to orient the probe 120 in a line that indicates the direction with the shortest distance towards the marker 10.

The signal processor 140 may be operatively connected to user interfaces such as keyboard, mouse, track-ball, buttons, touchscreen etc. The signal processor 140 may also include memory sources, connectors, cables, power sources, processors, programmable devices, analog-to-digital converters, digital-to-analog converters, buzzers, displays, LEDs, shielding.

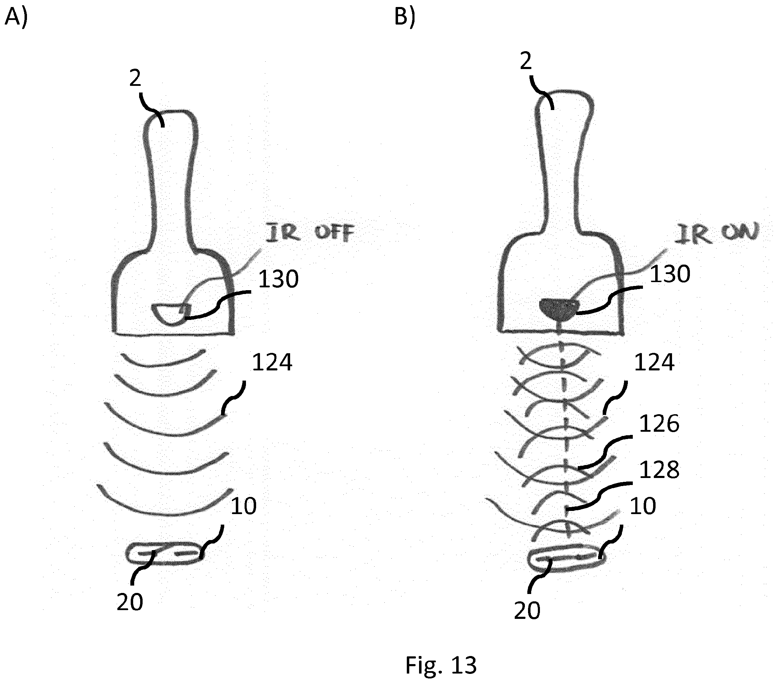

If infrared light strikes the photosensitive element 18 the switch 20 is activated and causes the piezo element 14.2 to emit specific ultrasound signals towards the probe 120. Figure 13A shows the switch 20 in open configuration when infrared light 128 is absent and the marker is not transmitting any specific ultrasound signals 18 or a characteristic backscatter, while Figure 13B shows the switch 20 in its closed state when the infrared light 128 is present and the marker is transmitting specific ultrasound signals 126 or a characteristic backscatter.

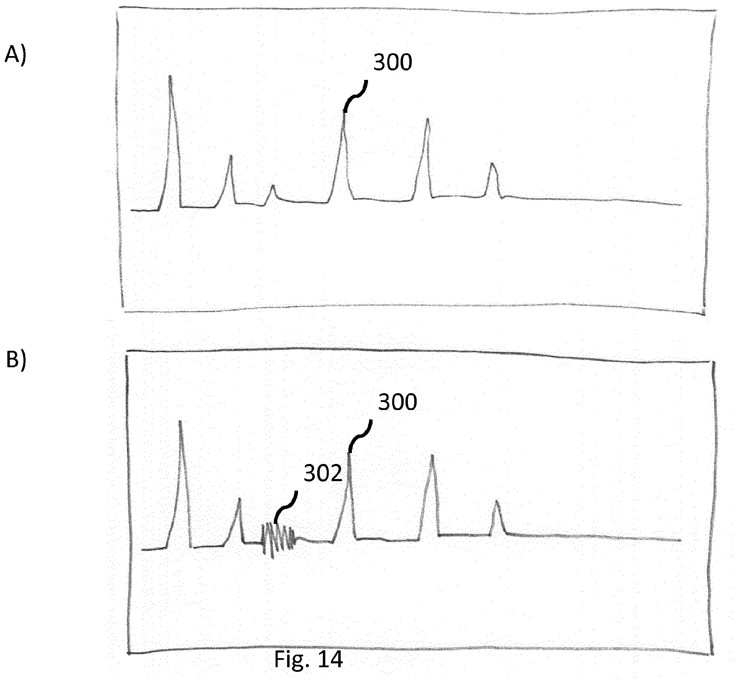

By changing the switch 20 from the open state to the closed state one can substantially change the received signal 130 by the probe 120. It may be that the received signal 300 by the probe 120 will then contain a short-term reflection 302 from the marker 110 as shown in Figure 14B or the whole received signal 130 might contain a backscatter noise 304 as shown in Figure 15B.

If the received signal 300 contains a short-term reflection 302 than this reflection may be used to calculate the depth of the marker 110 by evaluating the time between activation of the light source 130 and the reception of the specific reflection 302. By knowing the speed of sound in tissue (approx. 1540 m/s) and by knowing the specific delays within the marker 110 one can then calculate the distance of the marker 110 from the probe 120.

If the received signal 300 contains a backscatter noise 304 than other feature of the received signal 300 or of the ultrasound image 162 may be used to evaluate the position of the marker 110. The algorithms used to evaluate the received signal 300 or the ultrasound image 162 may include correlation, convolution, machine learning, deep learning, image recognition algorithms and signal analysis processes.

As shown in Figure 10 incident ultrasound waves 124 are not coherent and with increasing distance from the probe the incident ultrasound waves 124 might affect more markers 110 within the implanted region. If the markers 110 would not have the functionality to be switched on and of it may be difficult to differentiate between the markers 110 and decide which might lay directly in front of the probe 120. Therefore, the switching on and off of the marker 110 by another source of energy e.g. a light source is provided according to the present invention. Infra-red light with the wavelength between 800 nm and 950 nm might be a good source of energy as it penetrates the tissue into desired depth and remains relatively coherent. Therefore, directional specificity of the probe 120 is provided even if a marker 110 may be placed deeper in the tissue. Other energy sources might be included in the probe to activate the marker additionally or instead of infra-red light in order to activate the switch 20 in the marker 110. Accordingly, the energy converter (for instance piezo element 14.1) in the marker 110 would be adjusted accordingly as to be able to react to the energy pulses emitted from the probe 120.

Some of the challenges involved in detecting markers implanted within breast tissue (or elsewhere in a patient's body) include the relatively small cross-section of such marker in respect to the overall area of the body. This can be overcome by imaging first the respective region with ultrasound or mammography and narrowing down the area for further precise search (e.g. limiting the search only to one quadrant of the breast). Also, the scattering caused by the tissue inhomogeneity may present a complication.

In order to use handheld probe 120, a distal end of handheld probe 120 is put in contact with for instance the skin of a patients breast as shown in Figure 10. The distal end of handheld probe 120 is provided with a number of ultrasound transduces 122 and a light source 130 as shown if Figure 11. Handheld probe 120 may further comprise a push button 132 for switching the light source 130 on.

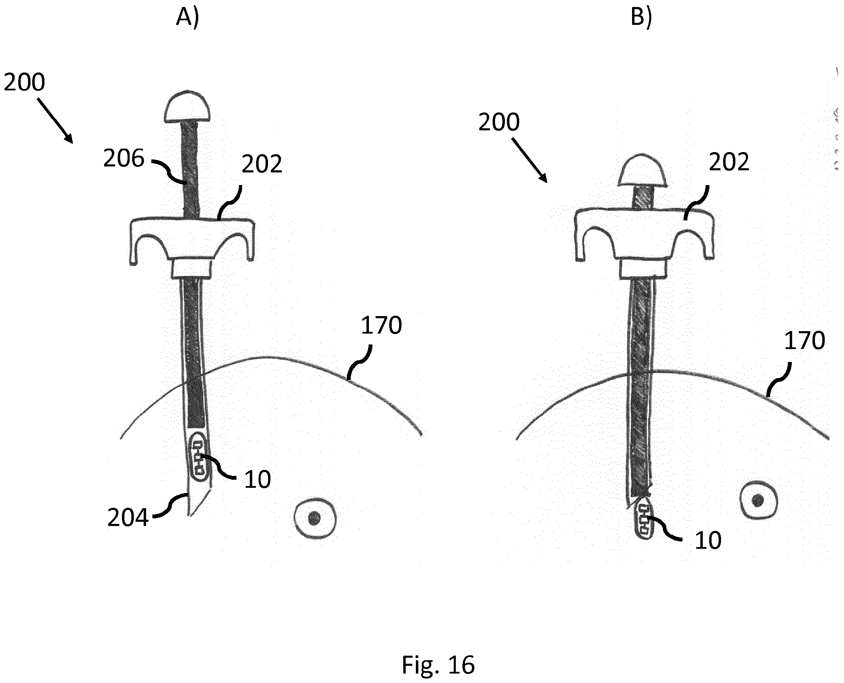

In Figure 16 it is illustrated how an ultrasound marker 110 can be implanted into body tissue by means of a delivery device 200 comprising a handpiece 202 and a hollow needle 204. Within the hollow needle 204, a pusher 206 is slidingly arranged. The hollow needle (cannula) 204 preferably has a sharp tip that facilitates puncturing body tissue. Within the hollow needle closed to its tip the ultrasound marker 10 is arranged prior to implantation. If the hollow needle 204 is inserted into body tissue, the ultrasound marker 10 can be pushed out the hollow needle 204 by means of pusher 206.

Instead of a sharp tip, the hollow needle may have a blunt tip to introduce the hollow needle trough a sheath or a coaxial or generally through another access path previously placed in the tissue. Alternatively, it is possible that the ultrasound marker 10 is placed in the tissue during open surgery.

Further, the hollow needle 204 of the delivery device 200 may carry a plurality of ultrasound markers 10. Accordingly, different ultrasound markers 10 can be placed on different targets within the patient's body by using the delivery device 200 a couple of times. Medical imaging may be used to confirm the location of the marker 10 relative to the target region.
- 10: ultrasound marker
- 12: enclosure
- 14.1, 14.2: piezo elements
- 16: trigger
- 18: photosensitive element
- 20: switch / field effect transistor
- 22: electrostatic discharge protection element
- 24: wire
- 26: capacitor
- 28: Schottky diode
- 30: resistor
- 32: protection resistor
- 34: mesh / fixation element
- 36: small elements
- 100: marker location system
- 110: marker
- 120: handheld probe
- 122: ultrasound transceiver
- 124: incident ultrasound waves
- 126: characteristic ultrasound waves / characteristic backscatter
- 128: infrared light/ trigger energy
- 130: trigger (light) source
- 132: trigger (light) source switch
- 140: signal processor
- 150: signal generator
- 152: speaker
- 160: display
- 162: ultrasound image
- 164: distance value
- 166: location marker signal
- 200: delivery device
- 202: handpiece
- 204: hollow needle
- 206: pusher
- 300: received ultrasound signal
- 302: short-term reflection
- 304: backscatter noise

## Claims

1. Ultrasound marker (10) comprising
- an enclosure (12)
- an energy transducer (14.1) for transducing impeding sound energy into electric energy
- an ultrasound emitter (14.2) that is configured to generate an ultrasound signal when fed with electric energy
- a trigger (16) that is operatively connected to the ultrasound emitter and that can be activated via a wireless signal, said trigger (16) being configured to control the generation of ultrasound by the ultrasound emitter in dependence of the wireless signal,
wherein the trigger (16) comprises a switch (20) that is operatively connected to the ultrasound emitter (14.2) and is configured to switch the ultrasound emitter (14.2) on or off, respectively, and
**characterized in that** the trigger (16) further comprises a photosensitive element (18) that is operatively connected to the switch (20) and is configured to control the switch (20) in when exposed to a light signal.

2. Marker (110) according to claim 1, further comprising an electric energy storage (26) that is connected to the energy transducer (14.1), said electric energy storage (26) being configured to store electric energy generated by the energy transducer.

3. Marker (110) according to at least one of claims 1 or 2, wherein the ultrasound emitter comprises a piezo element (14.2) and/or a capacitive micromachined ultrasonic transducer (CMUT).

4. Marker (110) according to at least one of claims 1 to 3, wherein the energy transducer comprises at least one of a piezo element (14.1), a capacitive micromachined ultrasonic transducer, a RC-resonance circuit and/ a RLC-resonance circuit.

5. Marker (110) according to at least one of claims 1 to 4, wherein the enclosure (12) encloses small elements (36) that are configured to amplify, modulate and/or filter the ultrasound waves (124) generated by the ultrasound emitter.

6. Marker (110) according to at least one of claims 1 to 5, further comprising fixation means that are configured to prevent dislocation of marker (110) after implantation.

7. Marker (110) according to at least one of claims 1 to 6, further comprising an ultra-sound resonator.

8. Marker (110) according to at least one of claims 1 to 7, wherein the marker (110) has a cross-section between 0.5 mm and 10 mm and a length between 2 mm and 30 mm.

9. Marker locating system (100) comprising
- an ultrasound marker (10) according to at least one of claim 1 to 8
- a handheld probe (120) that comprises an ultrasound transducer and is configured to emit and receive ultrasound
- a light source (130)
- a signal processor (140) for processing signals provided by the ultrasound transducer in response to receiving ultrasound signals
and
- a user guiding signal generator (150) that is configured to generate a user perceptible guiding signal in dependence of processed signals.

10. Marker locating system (100) according to claim 9, wherein the signal processor (140) comprises a trained classifier.

11. Marker locating system (100) according to claim 9 or 10, further comprising imaging means and a display (160).

12. Marker locating system (100) according to claim 11, wherein signal processor (140) is configured to generate a location marker signal (166) in a displayed image (162), the location marker signal (166) indicating the location of the marker (110).

13. Method for operating a marker locating system (100) according to one of claims 9 to 12, said method comprising the steps of:
- emitting energy to the marker (110);
- receiving energy by the marker (110)
- converting energy into electric energy in the marker (110)
- storing the electric energy in the marker (110)
- triggering switch (20) and activating ultrasound emitter in the marker (110) upon reception of a trigger signal received by the marker (110);
- emitting an ultrasound signal by the marker (110);
- receiving the ultrasound signal by the probe (120); and
- determining the distance between the marker (110) and the probe (120) based on ultrasound signal received by the probe (120).

## Patentansprüche

1. Ultraschallmarker (10) mit
- einem Gehäuse (12)
- einem Energiewandler (14.1) zum Umwandeln von eintreffender Schallenergie in elektrische Energie
- einem Ultraschallsender (14.2), der so konfiguriert ist, dass er ein Ultraschallsignal erzeugt, wenn er mit elektrischer Energie versorgt wird
- einem Auslöser (16), der mit dem Ultraschallsenderwirkverbunden ist und der über ein drahtloses Signal aktiviert werden kann, wobei der Auslöser (16) so konfiguriert ist, dass er das Erzeugen von Ultraschall durch den Ultraschallsender in Abhängigkeit von dem drahtlosen Signal steuert,
wobei der Auslöser (16) einen Schalter (20) umfasst, der mit dem Ultraschallsender (14.2) wirkverbunden und so konfiguriert ist, dass er den Ultraschallsender (14.2) ein- bzw. ausschaltet, und
**dadurch gekennzeichnet ist, dass**
der Auslöser (16) ferner ein lichtempfindliches Element (18) umfasst, das mit dem Schalter (20) wirkverbunden und so konfiguriert ist, dass es den Schalter (20) steuert, wenn er einem Lichtsignal ausgesetzt wird.

2. Marker (110) nach Anspruch 1, ferner umfassend einen elektrischen Energiespeicher (26), der mit dem Energiewandler (14.1) verbunden ist, wobei der elektrische Energiespeicher (26) so konfiguriert ist, dass er die vom Energiewandler erzeugte elektrische Energie speichert.

3. Marker (110) nach mindestens einem der Ansprüche 1 oder 2, wobei der Ultraschallsender ein Piezoelement (14.2) und/odereinen kapazitiven mikrobearbeiteten Ultraschallwandler (CMUT) umfasst.

4. Marker (110) nach mindestens einem der Ansprüche 1 bis 3, wobei der Energiewandler mindestens eines der folgenden Elemente umfasst: ein Piezoelement (14.1), einen kapazitiven mikromechanischen Ultraschallwandler, einen RC-Resonanzkreis und/oder einen RLC-Resonanzkreis.

5. Marker (110) nach mindestens einem der Ansprüche 1 bis 4, wobei das Gehäuse (12) kleine Elemente (36) einschließt, die so konfiguriert sind, dass sie die von dem Ultraschallsender erzeugten Ultraschallwellen (124) verstärken, modulieren und/oder filtern.

6. Marker (110) nach mindestens einem der Ansprüche 1 bis 5, ferner umfassend Fixationsmittel, die so konfiguriert sind, dass sie eine Dislokation des Markers (110) nach der Implantation verhindern.

7. Marker (110) nach mindestens einem der Ansprüche 1 bis 6, ferner umfassend einen Ultraschallresonator.

8. Marker (110) nach mindestens einem der Ansprüche 1 bis 7, wobei der Marker (110) einen Querschnitt zwischen 0,5 mm und 10 mm und eine Länge zwischen 2 mm und 30 mm aufweist.

9. Markerortungssystem (100) mit
- einem Ultraschallmarker (10) nach mindestens einem der Ansprüche 1 bis 8
- einer handhaltbaren Sonde (120), die einen Ultraschallwandler umfasst und so konfiguriert ist, dass sie Ultraschall aussendet und empfängt
- einer Lichtquelle (130)
- einem Signalprozessor (140) zum Verarbeiten von Signalen, die von dem Ultraschallwandler als Reaktion auf den Empfang von Ultraschallsignalen geliefert werden
und
- einem Benutzerführungssignalgenerator (150), der so konfiguriert ist, dass er ein für den Benutzer wahrnehmbares Führungssignal in Abhängigkeit von verarbeiteten Signalen erzeugt.

10. Markerortungssystem (100) nach Anspruch 9, wobei der Signalprozessor (140) einen trainierten Klassifikator umfasst.

11. Markerortungssystem (100) nach Anspruch 9 oder 10, das ferner Bildgebungsmittel und eine Anzeige (160) umfasst.

12. Markerortungssystem (100) nach Anspruch 11, wobei der Signalprozessor (140) so konfiguriert ist, dass er ein Ortsmarkierungssignal (166) in einem angezeigten Bild (162) erzeugt, wobei das Ortsmarkierungssignal (166) den Ort des Markers (110) anzeigt.

13. Verfahren zum Betreiben eines Markerortungssystems (100) nach einem der Ansprüche 9 bis 12, wobei das Verfahren die folgenden Schritte umfasst:
- Abgeben von Energie an den Marker (110);
- Empfangen von Energie durch den Marker (110)
- Umwandeln von Energie in elektrische Energie im Marker (110)
- Speichern der elektrischen Energie im Marker (110)
- Auslösen des Schalters (20) und Aktivieren des Ultraschallsenders in dem Marker (110) bei Empfang eines von dem Marker (110) empfangenen Auslösesignals;
- Aussenden eines Ultraschallsignals durch den Marker (110);
- Empfangen des Ultraschallsignals durch die Sonde (120); und
- Bestimmen der Entfernung zwischen dem Marker (110) und der Sonde (120) auf der Grundlage des von der Sonde (120) empfangenen Ultraschallsignals.

## Revendications

1. Marqueur (10) d'ultrasons comprenant
- un enceinte (12)
- un transducteur (14.1) d'énergie pour transformer de l'énergie acoustique gênante en énergie électrique
- un émetteur (14.2) d'ultrasons, qui est configuré pour créer un signal ultrasonore, lorsqu'il est alimenté en énergie électrique
- un déclencheur (16), qui est connecté fonctionnellement à l'émetteur d'ultrasons et qui peut être activé par un signal sans fil, ledit déclencheur (16) étant configuré pour commander la création d'ultrasons par l'émetteur d'ultrasons en fonction du signal sans fil,
dans lequel le déclencheur (16) comprend un interrupteur (20), qui est connecté fonctionnellement à l'émetteur (14.2) d'ultrasons et qui est configuré pour mettre l'émetteur (14.2) d'ultrasons en circuit ou hors circuit respectivement, et
**caractérisé en ce que** le déclencheur (16) comprend en outre un élément (18) photosensible, qui est connecté fonctionnellement à l'interrupteur (20) et qui est configuré pour ferme l'interrupteur (20), lorsqu'il est soumis à un signal lumineux.

2. Marqueur (110) suivant la revendication 1, comprenant en outre une accumulation (26) d'énergie, qui est connectée au transducteur (14.1) d'énergie, ladite accumulation (26) d'énergie étant configurée pour accumuler de l'énergie créée par le transducteur d'énergie.

3. Marqueur (110) suivant au moins l'une des revendications 1 ou 2, dans lequel l'émetteur d'ultrasons comprend un élément (14.2) piézoélectrique et/ou un transducteur (CMUT) d'ultrasons capacitif microusiné.

4. Marqueur (110) suivant au moins l'une des revendications 1 à 3, dans lequel le transistor d'énergie comprend au moins un élément (14.1) piézoélectrique, un transducteur d'ultrasons capacitif microusiné, un circuit à résonnance RC et un circuit à résonnance RLC.

5. Marqueur (110) suivant au moins l'une des revendications 1 à 4, dans lequel l'enceinte (12) renferme de petits éléments (36), qui sont configurés pour amplifier, moduler et/ou filtrer les ondes (124) ultrasonores créées par l'émetteur d'ultrasons.

6. Marqueur (110) suivant au moins l'une des revendications 1 à 5, comprenant en outre des moyens de fixation, qui sont configurés pour empêcher une dislocation du marqueur (110) après implantation.

7. Marqueur (110) suivant au moins l'une des revendications 1 à 6, comprenant en outre un résonnateur d'ultrasons.

8. Marqueur (110) suivant au moins l'une des revendications 1 à 7, dans lequel le marqueur (110) a une section transversale comprise entre 0,5 mm et 10 mm et une longueur comprise entre 2 mm et 30 mm.

9. Système (100) de localisation à marqueur comprenant
- un marqueur (10) d'ultrasons suivant au moins l'une des revendications 1 à 8
- une sonde (120) tenue à la main, qui comprend un transducteur d'ultrasons et est configurée pour émettre et recevoir des ultrasons
- une source (130) lumineuse,
- un processeur (140) de signal pour traiter des signaux donnés par le transducteur d'ultrasons en réaction à la réception de signaux ultrasonores
et
- un générateur (150) de signal de guidage d'un utilisateur, qui est configuré pour créer un signal de guidage perceptible par un utilisateur en fonction de signaux traités.

10. Système (100) de localisation à marqueur suivant la revendication 9, dans lequel le processeur (140) de signal comprend un classificateur ayant subi un apprentissage.

11. Système (100) de localisation à marqueur suivant la revendication 9 ou 10, comprenant en outre des moyens d'imagerie et un affichage (160).

12. Système (100) de localisation à marqueur suivant la revendication 11, dans lequel le processeur (140) de signal est configuré pour créer un signal (166) de marqueur de localisation dans une image (162) affichée, le signal (166) de marqueur de localisation indiquant la localisation du marqueur (110).

13. Procédé pour faire fonctionner un système (100) de localisation à marqueur suivant l'une des revendications 9 à 12, ledit procédé comprenant les stades de:
- émission d'énergie vers le marqueur (110) ;
- réception d'énergie par le marqueur (110)
- transformation de l'énergie en énergie électrique dans le marqueur (110)
- accumulation de l'énergie électrique dans le marqueur (110)
- déclenchement d'un interrupteur (20) et activation d'un émetteur d'ultrasons dans le marqueur (110), après réception d'un signal de déclenchement reçu par le marqueur (110);
- émission d'un signal ultrasonore par le marqueur (110);
- réception du signal ultrasonore par la sonde (120); et
- détermination de la distance entre le marqueur (110) et la sonde (120) sur la base du signal ultrasonore reçu par la sonde (120).
